# EUROPÄISCHE PATENTSCHRIFT

(11) **EP 2 340 794 B1**
(45) Veröffentlichungstag und Bekanntmachung des Hinweises auf die Patenterteilung: **23.10.2013**
(21) Anmeldenummer: 09016074.8
(22) Anmeldetag: 29.12.2009
(51) Int. Cl.: A61F 13/15, A61F 13/56, A61F 13/62

(54) **Inkontinenzartikel**
Incontinence article
Article pour incontinence

(43) Veröffentlichungstag der Anmeldung: 06.07.2011
(73) Patentinhaber: Nordenia Deutschland Gronau GmbH, 48599 Gronau (DE)
(72) Erfinder: Schönbeck Marcus, 33775 Versmold (DE)
(74) Vertreter: Lorenz, Bernd Ingo Thaddeus

(56) Entgegenhaltungen:
- WO-A1-2007/149016
- DE-A1-102004 053 469

## Beschreibung

Die Erfindung betrifft einen Inkontinenzartikel, insbesondere eine Inkontinenzvorlage für Erwachsene,
mit einem saugfähigen Grundkörper umfassend eine Innenseite mit einer flüssigkeitsdurchlässigen Innenlage, eine Außenseite mit einer flüssigkeitsdichten Außenlage sowie eine zwischen Innenlage und Außenlage angeordnete Saugeinlage und,
mit zwei Fixierabschnitten, die sich ausgehend von einem hinteren Taillenbereich seitlich von dem Grundkörper wegerstrecken und eine Außenschicht aus einem wasserstrahlverfestigten Vliesstoff aufweisen,
wobei die Fixierbandabschnitte zur Bildung eines den Inkontinenzartikel tragenden Gürtels miteinander verbindbar sind und wobei an einem vorderen Taillenbereich des Grundkörpers Hakenelemente angeordnet sind, die mit der Außenschicht ein Klettverschlusssystem bilden.

Bei Inkontinenzartikeln für Erwachsene ist es anders als bei Babywindeln im besonderen Maße erwünscht, dass diese möglichst nicht sichtbar sind und entsprechend unter der Kleidung nicht auftragen. So ergibt sich in der Regel auch ein anderer Schnitt als bei Babywindeln, wobei die Saugeinlage sich hauptsächlich im Schrittbereich erstreckt und wobei der vordere Taillenbereich, der hintere Taillenbereich sowie die Seiten des Inkontinenzartikels möglichst flach gehalten werden. Während bei Babywindeln häufig Windelohren vorgesehen sind, welche seitlich die Hüfte des Trägers umgreifen, ist bei einer gattungsgemäßen Inkontinerizvorlage für Erwachsene nur ein vergleichsweise schmaler tragender Gürtel vorgesehen.

Unterschiede ergeben sich auch beim Anlegen einer Babywindel einerseits und einer Inkontinenzvorlage für Erwachsene andererseits. So werden Babywindeln üblicherweise im Liegen angelegt, wobei erst der hintere Taillenbereich unter den Körper des Babys geschoben und der vordere Taillenbereich auf den Bauch aufgelegt werden, bevor die seitlichen Windelohren außen an dem vorderen Taillenbereich befestigt werden. Selbst wenn sich ein Baby beim Wickeln bewegt, kann es noch vergleichsweise leicht angehoben und festgehalten werden. Bei Inkontinenzartikeln für Erwachsene besteht das Problem, dass diese möglichst im Liegen, Sitzen oder Stehen anlegbar sein sollen. Das Anlegen soll sowohl durch den Benutzer selbst als auch durch eine Hilfsperson möglich sein. Zusätzlich ist aufgrund des höheren Gewichtes des Benutzers auch eine besonders leichte Handhabung notwendig. Entsprechend ist vorgesehen, mit den seitlich von dem Grundkörper abstehenden Fixierabschnitten einen umlaufenden, den Inkontinenzschutz tragenden Gürtel zu bilden, so dass der gesamte Inkontinenzartikel sich dann nicht mehr vollständig lösen kann, auch wenn der Benutzer bei dem Anlegen steht oder sich bewegt. Nachfolgend wird dann der vordere Taillenbereich nach oben geklappt, bis die an dem vorderen Taillenbereich vorgesehene Hakenelemente nach Art eines Klettverschlusses an der Außenschicht der Fixierbandabschnitte befestigt werden.

Zusätzlich ist zu berücksichtigen, dass bei Inkontinenzvorlagen für Erwachsene eine weitgehende Anpassung an unterschiedliche Körperproportionen möglich sein soll. Derartige Inkontinenzartikel, ähnlich wie bei Babywindeln erforderlich, in verschiedenen Größen bereitzuhalten ist aufwendig. Eine Unterscheidung verschiedener Produkte erfolgt in der Praxis häufig nur nach dem jeweiligen Aufnahmevermögen.

Die Begriffe innen und außen beziehen sich im Rahmen der vorliegenden Erfindung auf die Anordnung der Lagen und Schichten während des Tragens des Inkontinenzartikels durch einen Benutzer.

Aus der DE 10 2004 053 469 A1 ist ein Inkontinenzartikel mit den eingangs beschriebenen Merkmalen bekannt, wobei mit Wasserstrahlen verfestigter Vliesstoff lediglich im Rahmen einer Auflistung verschiedener Vliesstoffmaterialien genannt wird. Das Vliesstoffmaterial weist zur guten Verbindung mit Kletthaken inselartig angeordnete Bereich auf, die von gebundenen, d. h. verfestigten Strukturen begrenzt sind. Zur Verfestigung wird eine Thermoprägung, insbesondere eine Kalanderprägung oder ein Ultraschallschweißen offenbart. In den gebundenen Bereichen werden damit die Fasern des Vliesstoffes miteinander verschmolzen, wodurch das Material versteift wird und entsprechend seine Dehnbarkeit verliert. Abgesehen von den lediglich inselartig angeordneten ungebundenen Bereichen erstrecken sich die gebundenen Konturen über das gesamte Vliesstoffmaterial, so dass ein davon abgeschnittener Streifen in seiner Längsrichtung eine hohe Zugfestigkeit und Steifigkeit aufweist.

aus der WO 2007/149016 A1 ist ein Inkontinenzartikel für Erwachsene bekannt, der gemäß dem üblichen Aufbau einen saugfähigen Grundkörper umfassend eine Innenseite mit einer flüssigkeitdurchlässigen Innenlage, eine Außenseite mit einer flüssigkeitsdichten Außenlage sowie eine zwischen Innenlage und Außenlage angeordnete Saugeinlage aufweist. Des Weiteren sind zwei Fixierabschnitte vorgesehen, die sich ausgehend von einem hinteren Taillenbereich seitlich von dem Grundkörper wegerstrecken und eine Außenschicht aus Fliesstoff aufweisen, wobei die Fixierabschnitte zur Bildung eines den Inkontinenzartikel tragenden Gürtels miteinander verbindbar sind. Die Fixierbandabschnitte sind steif. Eine Elastizität des insgesamt gebildeten Gürtels wird dadurch erreicht, dass an dem Grundkörper eine elastische Folie eingebunden wird. Die Herstellung des Grundkörpers ist deshalb aufwendig.

Der vorliegenden Erfindung liegt die Aufgabe zugrunde, eine Inkontinenzschutz mit den eingangs beschriebenen Merkmalen anzugeben, der bei einer kostengünstigen Herstellung eine hohe Sicherheit gegen einen versehentliches Öffnen gewährleistet.

Diese Aufgabe durch einen Inkontinenzartikel gemäß Patentanspruch 1 gelöst. Erfindungsgemäß ist ein elastischer Bereich an einem der Fixierbandabschnitte ausgebildet, wobei die Außenschicht in dem elastischen Bereich mit einer elastischen Schicht verbunden ist. Die elastische Schicht, üblicherweise eine Folie mit einer Dicke zwischen 40 und 150 µm, bewirkt nach einer Dehnung eine elastische Rückstellung. Um insgesamt eine ausreichende Elastizität und damit einen höheren Tragekomfort sicherzustellen, sind bevorzugt beide Fixierbandabschnitte mit einem elastischen Bereich versehen.

Der mit Wasserstrahlen verfestigte Vliesstoff wird auch als Spunlace-Nonwoven bezeichnet. Durch die Wasserstrahlverfestigung wird eine schlaufenförmige Faserstruktur erzeugt, welche zu einer höheren Stabilität des Materials beiträgt. Überraschenderweise kann sich der mit Wasserstrahlen verfestigte Vliesstoff auch trotz der Verfestigung sehr gut mit Hakenelementen verbinden, wodurch ein zuverlässiges Klettverschlusssystem bereitgestellt wird. Spunbond-Vliesstoffe sind in der Regel auch durch die Ausbildung eines zellenförmigen Musters erkennbar, welches Bereiche hoher Faserdichte und geringer Faserdichte aufweist. Wie nachfolgend noch hervorgehoben, zeichnet sich der durch Wasserstrahlen verfestigte Vliesstoff auch durch eine gewisse Dehnbarkeit aus, was gerade für die Bereitstellung elastischer Bereiche von Vorteil ist.

Im Rahmen der Erfindung sind die Fixierbandabschnitte entweder die Enden eines durchgehenden Fixierbandes oder als voneinander separate Stücke seitlich an dem Grundkörper befestigt, wobei dann der den Inkontinenzschutz tragende Gürtel von den beiden Stücken sowie dem dazwischen liegenden hinteren Taillenbereich gebildet wird. Unabhängig davon, ob die Fixierbandabschnitte separater Stücke oder Enden eines durchgängigen Bandes sind, kann der zumindest eine elastische Bereich im hinteren Taillenbereich und/oder an zumindest einem der beiden Fixierabschnitte vorgesehen sein.

Die elastische Schicht ist bevorzugt zwischen der zuvor beschriebenen Außenschicht aus dem mit Wasserstrahlen verfestigten Vliesstoff und einer Innenschicht aus Vliesstoff, welche bei einem angelegten Inkontinenzschutz an dem Körper des Benutzers anliegt, einkaschiert. Die Innenschicht kann dabei aus einem anderen Vliesmaterial als die Außenschicht gebildet sein. Besondere Vorteile ergeben sich jedoch, wenn auch die Innenschicht aus einem mit Wasserstrahlen verfestigten Vliesstoff gebildet ist, wobei dann die Innenschicht und die Außenschicht gleichermaßen dehnbar sind. Wenn außer der nur bereichsweise dazwischen vorgesehenen elastischen Schicht keine weiteren Zwischenschichten vorgesehen sind, ergibt sich der Vorteil, dass das gebildete bereichsweise zweischichtige und bereichsweise dreischichtige Material des Fixierbandabschnittes bzw. der Fixierbandabschnitte ohne eine weitere Vorbehandlung dehnbar und in den elastischen Bereichen elastisch dehnbar ist. Es ist damit nicht erforderlich, die außen liegenden Vliesstoffschichten bei der Herstellung des Fixierbandabschnittes auf eine unter Zugspannung stehende elastische Schicht aufzubringen (Stretch-bond-Verfahren) oder das gebildete Material einer Aktivierung durch eine Vorverstreckung zu unterziehen. Zumindest vor einem erstmaligen Gebrauch des Inkontinenzartikels kann damit erreicht werden, dass die Außenschicht und die Innenschicht in dem elastischen Bereich plan und ohne Aufwerfungen an der elastischen Schicht anliegen. Wenn sowohl die Innenschicht als auch die Außenschicht aus Spunlace-Nonwoven bestehen und bei der Benutzung eine zu starke nicht elastische Dehnung vermieden werden soll, kann auch außerhalb der elastischen Bereiche eine steife Schicht zwischen der Außenschicht und der Innenschicht einkaschiert werden.

Sofern in einer alternativen Ausgestaltung der Erfindung für die Innenschicht ein nicht dehnbares Vliesmaterial eingesetzt wird, erfolgt bevorzugt vor der Verbindung der Fixierbandabschnitte an dem Grundkörper eine Aktivierung der elastischen Bereiche mittels einer Vorverstreckung.

Das Flächengewicht der Außenschicht beträgt vorzugsweise zwischen 20 und 100 g/m². Sofern auch wie zuvor als bevorzugt beschrieben eine Innenschicht vorgesehen wird, beträgt das Flächengewicht abhängig von der Art des Vliesstoffmaterials zwischen 15 und 100 g/m². Im Rahmen der Erfindung ist als elastische Schicht vorzugsweise eine elastische Folie mit einer Dicke zwischen 40 µm und 150 µm vorgesehen. Im Rahmen der Erfindung wird die Folie gemäß ihrer Funktion zunächst lediglich als elastische Schicht angesehen. Die genaue Ausgestaltung der Folie ist dadurch jedoch nicht beschränkt. Neben einer Monofolie sollen auch Laminate und Coextrusionsfolien mit mehreren Folienschichten mit umfasst sein.

Die Fixierbandabschnitte sind bevorzugt als einfache gerade Streifen ausgebildet und können so auf besonders einfache Weise in Querrichtung von einer Materialbahn abgeschnitten werden. Wie bereits zuvor dargelegt, sollen die einen Gürtel bildenden Fixierbandabschnitte einen sicheren Halt und einen hohen Tragekomfort gewährleisten, wobei die Fixierbandabschnitte auch unter der Kleidung eines Benutzers möglichst nicht sichtbar sein sollen. Geeignet sind beispielsweise als gerade Streifen ausgebildete Fixierbandabschnitte mit einer Höhe zwischen 50 mm und 150 mm. Die Länge der Fixierbandabschnitte beträgt ohne Zugspannung vorzugsweise zumindest 200 mm. Die Länge kann beispielsweise zwischen 250 mm und 400 mm liegen. Dabei ist zu berücksichtigen, dass auch eine Anpassung an verschiedene Körperproportionen ermöglicht werden soll.

Bei dem Anlegen des Inkontinenzartikels wird zunächst der Grundkörper mit seinem hinteren Taillenbereich an den Benutzer angelegt, bevor dann die beiden Fixierbandabschnitte unter Bildung eines Gürtels um den Benutzer herumgeführt und miteinander verbunden werden. Zu diesem Zweck weist vorzugsweise einer der beiden Fixierbandabschnitte an seiner Innenschicht ein aufgesetztes Hakenelement auf, welches mit der Außenschicht des anderen Fixierbandabschnittes unter Bildung eines Klettverschlusses verbunden werden kann. Durch eine unterschiedlich weite Überlappung der beiden Fixierbandabschnitte ist eine Anpassung an unterschiedliche Körperumfänge sowie eine Einstellung der von dem Gürtel ausgeübten Kraft möglich. Die Fixierbandabschnitte weisen, bezogen auf den seitlichen Rand des Grundkörpers, ohne Zugbeanspruchung bevorzugt jeweils eine Länge von zumindest 200 mm auf. Die Länge kann beispielsweise zwischen 250 mm und 400 mm betragen.

Die an dem vorderen Taillenbereich des Grundkörpers befestigten Hakenelemente des Klettverschlusses sind vorzugsweise an der Innenseite des Grundkörpers angeordnet. Alternativ ist auch eine Befestigung an der Außenseite möglich, wobei dann die Hakenelemente den vorderen Taillenbereich seitlich oder am vorderen Rand übergreifen.

Das von der Außenschicht aus Spunlace-Nonwoven und den zugeordneten Hakenelementen gebildete Klettverschlusssystem zeichnet sich durch eine hohe und gleichmäßige Klettwirkung aus. Dies gilt sowohl für die bei einem Abziehen der Hakenelemente von der Außenschicht auftretenden Kräfte (Peel-Versuch) als auch bei Kräften, die parallel zur Kontaktfläche wirken (Shear-Versuch). Schließlich ergibt sich auch der Vorteil, dass das auch in einem gewissen Maße dehnbare Spunlace-Nonwoven mit geringen Fertigungskosten herstellbar ist.

Die Erfindung wird im Folgenden anhand einer lediglich ein Ausführungsbeispiel darstellenden Zeichnung erläutert. Es zeigen:

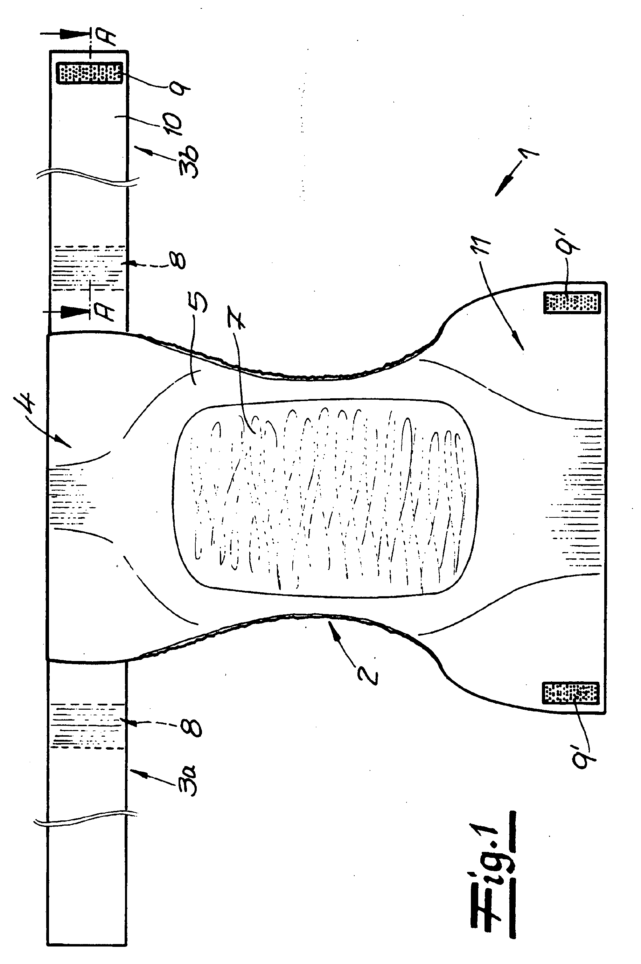

- **Fig. 1**: eine Draufsicht auf einen erfindungsgemäßen Inkontinenzartikel,
- **Fig. 2**: den Inkontinenzartikel gemäß Fig. 1 bei dem Anlegen und
- **Fig. 3**: einen Schnitt entlang der Linie A-A der Fig. 1.

Der erfindungsgemäße Inkontinenzartikel in Form einer Inkontinenzvorlage 1 für Erwachse umfasst gemäß seinem allgemeinen Aufbau einen Grundkörper 2 sowie Fixierbandabschnitte 3a, 3b auf, die sich ausgehend von einem hinteren Taillenbereich 4 seitlich von dem Grundkörper 2 wegerstrecken.

Der saugfähige Grundkörper 2 umfasst eine Innenseite 5 mit einer flüssigkeitsdurchlässigen Innenlage, einer Außenseite 6 mit einer flüssigkeitsdichten Außenlage sowie eine zwischen der Innenlage und der Außenlage angeordnete Saugeinlage 7 auf.

Gemäß dem in den Figuren dargestellten Ausführungsbeispiel sind die Fixierbandabschnitte 3a, 3b als separate Stücke bereitgestellt. Alternativ können die Fixierbandabschnitte 3a, 3b aber auch die Enden eines durchgehenden Fixierbandes sein.

Die Fixierbandabschnitte 3a, 3b weisen jeweils einen elastischen Bereich 8 auf. Zusätzlich ist an einem Fixierbandabschnitt 3b ein Hakenelement 9 an einer Innenschicht 10 angeordnet. Zusätzliche Hakenelemente 9' befinden sich an einem vorderen Taillenbereich 11 an der Innenseite 5 des Grundkörpers 2.

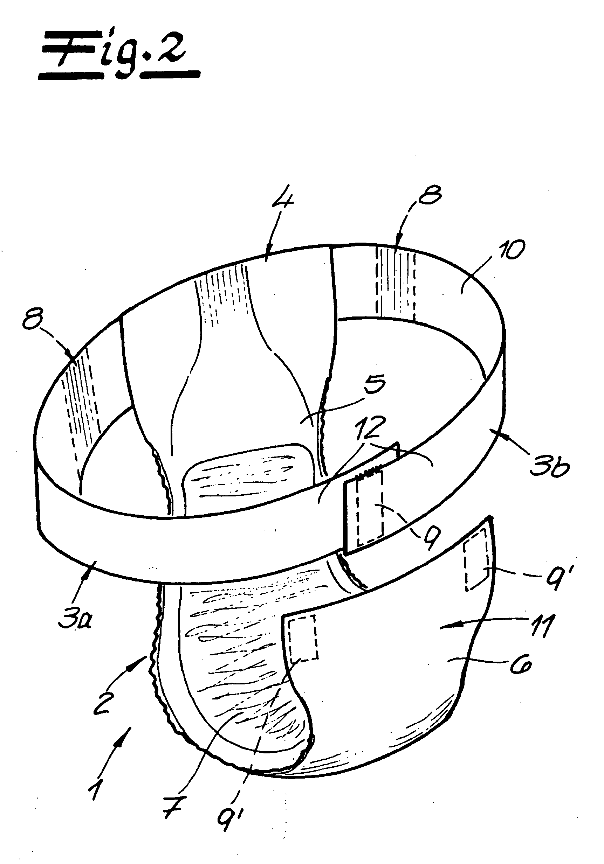

Aus der Fig. 2 ist ersichtlich, wie die Inkontinenzvorlage 1 an einem zum Zwecke der Übersichtlichkeit nicht dargestellten Benutzer anzulegen ist. Zunächst wird der hintere Taillenbereich 4 an den Benutzer angelegt, wobei dann die Fixierbandabschnitte 3a, 3b derart um die Taille gelegt werden, dass der an der Innenschicht 10 eines der Fixierbandabschnitte 3b angeordnete Hakenelemente 9 auf einer Außenschicht 12 des anderen Fixierbandabschnittes 3a zu liegen kommt, wobei das Hakenelement 9 mit der zugeordneten Außenschicht 12 ein Klettverschlusssystem bildet. Durch die Fixierbandabschnitte 3a, 3b und den dazwischen liegenden hinteren Taillenbereich 4 wird ein umlaufender Gürtel gebildet, der die Inkontinenzvorlage 1 trägt. Der vordere Taillenbereich 11 muss sodann lediglich zwischen den Beinen des Benutzers durchgeführt und an den Außenschichten 12 der Fixierbandabschnitte 3a, 3b über die Hakenelemente 9' befestigt werden. Es ergibt sich der Vorteil, dass die Inkontinenzvorlage 1 leicht angelegt werden kann, wenn der Benutzer liegt, steht oder auch sitzt. Der Hauptteil der Saugeinlage 7 ist im Schrittbereich angeordnet, so dass die Inkontinenzvorlage 1 nur wenig aufträgt.

Da die beiden Fixierbandabschnitte 3a, 3b mit unterschiedlicher Überlappung aufeinander gelegt werden können, ist eine Anpassung an einen unterschiedlichen Körperumfang sowie eine Einstellung der durch die elastischen Bereiche 8 ausgeübten Zugkraft möglich, so dass ein sowohl sicherer als auch komfortabler Sitz erreicht werden können.

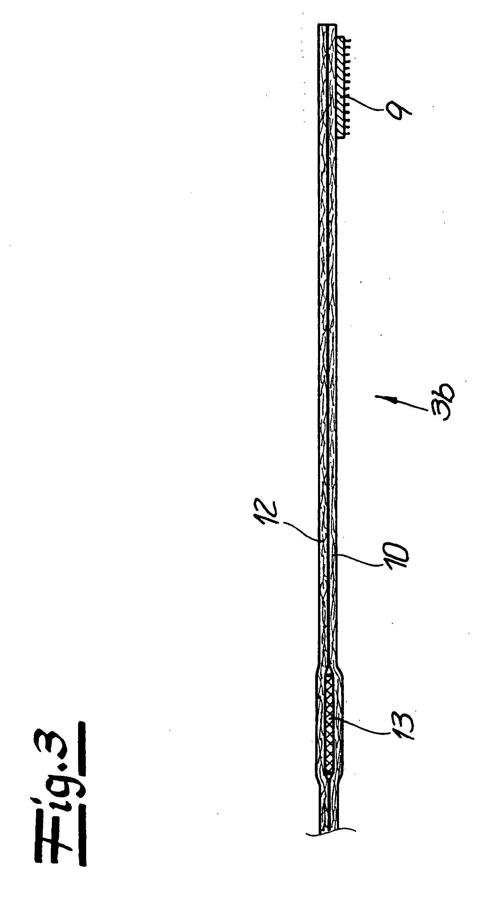

Die Fig. 3 zeigt in einer Schnittdarstellung die Ausgestaltung des mit dem Hakenelement 9 versehenen Fixierbandabschnittes 3b, wobei der andere Fixierbandabschnitt 3a bis auf die fehlende Anordnung eines Hakenelementes 9 gleich ausgebildet ist. Erfindungsgemäß ist die Außenschicht 12 der Fixierbandabschnitte 3a, 3b von einem mit Wasserstrahlen verfestigten Vliesstoff gebildet, der auch als Spunlace-Nonwoven bezeichnet wird. Überraschenderweise weist die entsprechend ausgebildete Außenschicht 12 eine sehr gute Fähigkeit auf, um sich mit den Hakenelementen 9, 9' nach Art eines Klettverschlusses zu verbinden.

In dem dargestellten Ausführungsbeispiel ist in dem elastischen Bereich 8 eine elastische Folie 13 mit einer Dicke von typischerweise zwischen 40 und 150 µm zwischen die Innenschicht 10 und die Außenschicht 12 einkaschiert. Des Weiteren ist gemäß dem dargestellten, bevorzugten Ausführungsbeispiel vorgesehen, dass auch die Innenschicht 10 von einem mit Wasserstrahlen verfestigten Vliesstoff gebildet ist. Da ein solches Spunlace-Nonwoven, welches beispielsweise mit einem Flächengewicht zwischen 20 und 100 g/m² vorgesehen sein kann, eine gewisse Dehnbarkeit aufweist, ist vor einem erstmaligen Gebrauch der Inkontinenzvorlage 1 eine aufwendige Aktivierung durch eine Vorreckung oder dergleichen nicht erforderlich. Vielmehr kann die Inkontinenzvorlage 1 so ausgeführt werden, dass zumindest vor einem erstmaligen Gebrauch die Außenschicht 12 und in dem dargestellten Ausführungsbeispiel auch die vorgesehene Innenschicht 10 plan und ohne Aufwerfungen an der elastischen Folie 13 anliegen.

Die Fixierbandabschnitte 3a, 3b sind als gerade Streifen ausgebildet und weisen eine Länge von mehr als 200 mm sowie eine Höhe zwischen 50 und 150 mm auf.

## Patentansprüche

1. Inkontinenzartikel, insbesondere Inkontinenzvorlage (1) für Erwachsene,
mit einem saugfähigen Grundkörper (2) umfassend eine Innenseite (5) mit einer flüssigkeitsdurchlässigen Innenlage, einer Außenseite (6) mit einer flüssigkeitsdichten Außenlage sowie eine zwischen Innenlage und Außenlage angeordnete Saugeinlage (7), und
mit zwei Fixierbandabschnitten (3a, 3b), die sich ausgehend von einem hinteren Taillenbereich (4) seitlich von dem Grundkörper (2) wegerstrecken und eine Außenschicht (12) aus einem mit Wasserstrahlen verfestigten Vliesstoff aufweisen,
wobei die Fixierbandabschnitte (3a, 3b) zur Bildung eines den Inkontinenzartikel tragenden Gürtels miteinander verbindbar sind und wobei an einem vorderen Taillenbereich (11) des Grundkörpers (2) Hakenelemente (9') angeordnet sind, die mit der Außenschicht (12) ein Klettverschlusssystem bilden, **dadurch gekennzeichnet, dass** zumindest ein elastischer Bereich (8) an einem Fixierbandabschnitt (3a, 3b) ausgebildet ist, wobei die Außenschicht (12) in dem elastischen Bereich (8) mit einer elastischen Schicht verbunden ist.

2. Inkontinenzartikel nach Anspruch 1, **dadurch gekennzeichnet, dass** die elastische Schicht zwischen der Außenschicht (12) und einer Innenschicht (10) aus Vliesstoff einkaschiert ist.

3. Inkontinenzartikel nach Anspruch 2, **dadurch gekennzeichnet, dass** die Innenschicht (10) von einem mit Wasserstrahlen verfestigten Vliesstoff gebildet ist.

4. Inkontinenzartikel nach einem der Ansprüche 1 bis 3, **dadurch gekennzeichnet, dass** die Außenschicht (12) in dem elastischen Bereich (8) plan und ohne Aufwerfungen an der elastischen Schicht anliegt.

5. Inkontinenzartikel nach Anspruch 2, **dadurch gekennzeichnet, dass** der zumindest eine elastische Bereich (8) durch eine Vorverstreckung aktiviert ist.

6. Inkontinenzartikel nach einem der Ansprüche 2 bis 5, **dadurch gekennzeichnet, dass** die Innenschicht (10) ein Flächengewicht zwischen 15 und 100 g/m² aufweist.

7. Inkontinenzartikel nach einem der Ansprüche 1 bis 6, **dadurch gekennzeichnet, dass** als elastische Schicht eine Folie (13) mit einer Dicke zwischen 40 und 150 µm vorgesehen ist.

8. Inkontinenzartikel nach einem der Ansprüche 1 bis 7, **dadurch gekennzeichnet, dass** die Außenschicht (12) ein Flächengewicht zwischen 20 und 100 g/m² aufweist.

9. Inkontinenzartikel nach einem der Ansprüche 1 bis 8, **dadurch gekennzeichnet, dass** die Fixierbandabschnitte (3a; 3b) als gerade Streifen ausgebildet sind.

10. Inkontinenzartikel nach Anspruch 9, **dadurch gekennzeichnet, dass** die Fixierbandabschnitte (3a, 3b) eine Höhe zwischen 50 und 150 mm aufweisen.

11. Inkontinenzartikel nach einem der Ansprüche 1 bis 10, **dadurch gekennzeichnet, dass** die Fixierbandabschnitte (3a, 3b) ohne eine Zugbeanspruchung jeweils eine Länge von mindestens 200 mm aufweisen.

12. Inkontinenzartikel nach einem der Ansprüche 1 bis 11, **dadurch gekennzeichnet, dass** die Hakenelemente (9') des Klettverschlusssystems an dem vorderen Taillenbereich (11) an der Innenseite (5) des Grundkörpers (2) angeordnet sind.

## Claims

1. An incontinence brief for an adult wearer, the brief comprising:
an absorbent body having a fluid-permeable inner side, a fluid-tight outer side, and an absorbent liner between the inner side and the outer side, the body being subdivided into a central part adapted to fit in a crotch region of the wearer, a rear part connected to the central part and adapted to fit against a rear waist region of the wearer, and a front part adapted to fit against a front waist region of the wearer;
two fastening straps each adapted to fit around a respective side of a waist of the wearer and having a rear end secured to the rear part and a front part, at least one of the fasteners being provided between its respective front and rear ends with an elastic layer forming an elastically stretchable region, each of the fasteners having an outer layer formed by a water-jet consolidated fleece;
a hook-and-barb fastener that can releasably secure the front ends of the straps together at the front waist region; and respective hook patches on the front part releasably securable to the outer layers of the fastening straps at the front waist region.

2. The incontinence brief defined in claim 1 wherein the one strap has an inner layer that, together with the respective outer layer, sandwiches the elastic layer.

3. The incontinence brief defined in claim 2 wherein the elastic layer is wholly contained between the respective inner and outer layers.

4. The incontinence brief defined in claim 2 wherein the inner layer is made of a water-jet consolidated fleece.

5. The incontinence brief defined in claim 2 wherein the elastic layer and inner and outer layers lie flatly against each other, the elastic layer being activated by prestretching.

6. The incontinence brief defined in claim wherein the inner layer has a weight per unit of area of between 15 g/m and 100 g/m².

7. The incontinence brief defined in claim 2 wherein the elastic layer is a film having a thickness of between 40 µm and 150 µm.

8. The incontinence brief defined in claim 2 wherein the outer layer has a weight per unit of area of between 20 g/m und 100 g/m .

9. The incontinence brief defined in claim 2 wherein the fastening straps are straight bands.

10. The incontinence brief defined in claim 9 wherein the fastening straps have a width of between 50 mm and 150 mm.

11. The incontinence brief defined in claim 2 wherein when not tensioned the fastening straps each have a length of at 3 least 200 mm.

12. The incontinence brief defined in claim 1 wherein the hook patches are provided at the front waist region on the inner side of the body.

## Revendications

1. Article pour incontinents, notamment couche pour incontinents (1) destinée aux adultes,
avec un corps de base (2) absorbant comprenant une face intérieure (5) avec une couche intérieure perméable aux liquides, une face extérieure (6) avec une couche extérieure imperméable aux liquides, ainsi qu'avec un insert absorbant (7), placé entre la couche intérieure et la couche extérieure et
avec deux sections de ruban de fixation (3a, 3b), qui en partant d'une zone de taille (4) arrière s'éloignent latéralement du corps de base (2) et qui comportent une couche extérieure (12) en un non-tissé consolidé par jets d'eau,
les sections de ruban de fixation (3a, 3b) pouvant être reliées l'une à l'autre pour former une ceinture portant l'article pour incontinents et sur une zone de taille (11) avant du corps de base (2) étant placés des éléments à crochets (9') qui forment avec la couche extérieure (12) un système de fermeture auto-agrippante, **caractérisé en ce qu'**au moins une zone élastique (8) est conçue sur une section de ruban de fixation (3a, 3b), dans la zone élastique (8), la couche extérieure (12) étant reliée avec une couche élastique.

2. Article pour incontinents selon la revendication 1, **caractérisé en ce que** la couche élastique est contrecollée entre la couche extérieure (12) et une couche intérieure (10) en non-tissé.

3. Article pour incontinents selon la revendication 2, **caractérisé en ce que** la couche intérieure (10) est formée par un non-tissé consolidé par jets d'eau.

4. Article pour incontinents selon l'une quelconque des revendications 1 à 3, **caractérisé en ce que** dans la zone élastique (8), la couche extérieure (12) est adjacente de manière plane et sans élévations à la couche élastique.

5. Article pour incontinents selon la revendication 2, **caractérisé en ce que** l'au moins une zone élastique (8) est activée par une pré-extension.

6. Article pour incontinents selon l'une quelconque des revendications 2 à 5, **caractérisé en ce que** la couche intérieure (10) présente un grammage compris entre 15 et 100 g/m².

7. Article pour incontinents selon l'une quelconque des revendications 1 à 6, **caractérisé en ce qu'**en tant que couche élastique, il est prévu un film (13) d'une épaisseur comprise entre 40 et 150 µm.

8. Article pour incontinents selon l'une quelconque des revendications 1 à 7, **caractérisé en ce que** la couche extérieure (12) présente un grammage compris entre 20 et 100 g/m².

9. Article pour incontinents selon l'une quelconque des revendications 1 à 8, **caractérisé en ce que** les sections de ruban de fixation (3a ; 3b) sont conçues en tant que bandes droites.

10. Article pour incontinents selon la revendication 9, **caractérisé en ce que** les sections de ruban de fixation (3a, 3b) présentent une hauteur comprise entre 50 et 150 mm.

11. Article pour incontinents selon l'une quelconque des revendications 1 à 10, **caractérisé en ce que** les sections de ruban de fixation (3a, 3b) présentent chacune une longueur d'au moins 200 mm, sans contrainte par traction.

12. Article pour incontinents selon l'une quelconque des revendications 1 à 11, **caractérisé en ce que** les éléments à crochets (9') du système de fermeture auto-agrippante sont placés sur la zone de taille (11) avant, sur la face intérieure (5) du corps de base (2).
